(19) Europäisches Patentamt
European Patent Office
Office européen des brevets

(11) Publication number: 0 448 181 A2

# (12) EUROPEAN PATENT APPLICATION

(21) Application number: **91200692.1**

(22) Date of filing: **25.03.91**

(51) Int. Cl.[5]: **A61K 37/02**, C07K 15/00, C12P 21/00, C07K 3/12, //(C12P21/00,C12R1:91)

(30) Priority: **23.03.90 EP 90200696**
**08.10.90 EP 90202674**

(43) Date of publication of application: **25.09.91 Bulletin 91/39**

(84) Designated Contracting States: **AT BE CH DE DK ES FR GB GR IT LI LU NL SE**

(71) Applicant: **GIST-BROCADES N.V.**
**Wateringseweg 1 P.O. Box 1**
**NL-2600 MA Delft(NL)**

(72) Inventor: **Luger, Thomas, Prof.Dr.med. c/o Klinik und Poliklinik f. Hautkrankheiten Universität Münster**
**Von-Esmarck-Strasse 56 W-4400 Münster(DE)**
Inventor: **Köck, Andreas, Dr. c/o University of Vienna**
**Department of Urology Alserstrasse 4**
**A-1090 Wien(AT)**

(74) Representative: **Huygens, Arthur Victor, Dr. et al**
**c/o Gist-Brocades N.V. Patent & Trademarks Department Wateringseweg 1 PO Box 1**
**NL-2600 MA Delft(NL)**

## (54) Interleukin-6 inhibiting compositions.

(57) A composition of matter having IL-6 inhibitory activity, said composition comprising a cell culture supernatant wherein the cells have been stimulated for production of IL-6 inhibitory activity, or partially purified or isolated IL-6 inhibitory components thereof are useful as antiinflammatory drugs.

EP 0 448 181 A2

## Technical Field

The present invention relates to therapeutic compositions which counteract the effects of the cytokine IL-6 and to methods for their preparation.

## Background Art

Cytokines are multifunctional soluble factors occurring in mammals that play an important role in the regulation of cellular functions such as the mediation of inflammatory and immune reactions. See, for instance, New England Journal of Medicine (1987) 317:940-945. These cytokines or regulatory factors are (glyco)proteins which bind to specific receptors and influence activation, proliferation and differentiation of both immune and non-immune cells. These hormone-like substances are released by lymphocytes, which explains the early name lymphokines, but also by other immune cells such as fibroblasts, endothelial cells, epithelial cells including keratinocytes and stroma cells. One of those cytokines is interleukin-6 (IL-6), which has been shown to be an important mediator in the initiation and the sustainment of inflammatory reactions. See, e.g., J. Immunol. (1989) 142:1922-1928 and Immunology Today (1987) 84: 137. IL-6 is also known under the names B-cell stimulatory factor-2, interferon β2, hybridoma growth factor, hepatocyte stimulating factor, macrophage granulocyte inducer-2 and 26 kDa protein. IL-6 is believed to be associated with diseases as psoriasis, allergic reactions, rheumatic afflictions and inflammatory diseases of the skin, the lung or the gastro-intestinal tract. The preparation and biochemical characterization of IL-6 are described in Sehgal, P.B. et al., J. Interferon Res. (1987) 7:521 and Van Damme, J. et al., J. Ex Med. (1987) 165:914.

The action of several cytokines is known to be balanced by antagonists, but until presently no natural antagonist of IL-6 activity has been found.

Although the substances described hereafter are denoted as inhibitors, it is not known whether the inhibiting effect is based on an inactivation of the IL-6 protein, on the blocking of the receptor site or on another interfering action. Therefore the use of the term "inhibitor" does not imply that the effects of the invention may not be caused by antagonism.

The present invention provides compositions which are IL-6 inhibitors, denoted herein with the abbreviation IL-6i. In particular, IL-6 inhibitory substances have been found which are of natural origin. One such interleukin-6 inhibitor is a protein having a molecular weight of approximately 10 kilodalton (kDa); it has no inhibitory effect on IL-1, IL-2 or IL-4. Another such interleukin-6 inhibitor is a protein having a molecular weight of approximately 44 kDa. Both IL-6i are produced by mammalian cells. These IL-6 inhibitors are antiinflammatory agents.

The medical treatment of inflammatory afflictions is still unsatisfactory. The most widely used medicines are compositions containing steroids, particularly corticosteroids. These have many undesirable side effects, especially when they are administered for a prolonged period, e.g., deterioration of the skin and inhibition of the adrenal cortex. The IL-6 inhibitors of the invention do not possess such side effects.

## Disclosure of the Invention

The invention provides compositions which inhibit the activity of IL-6 and are thus useful as antiinflammatory drugs. The IL-6 inhibitors of the invention can be derived from supernatants of cells which have been stimulated to produce the proteinaceous substances having IL-6i activity by a variety of regimes, including, for example, stimulation with UV light or phorbol myristate acetate (PMA). The supernatants exhibit IL-6 inhibitory activity, and the components responsible for this activity can be prepared in purified form from these supernatants.

These active components appear to be proteins, since after overnight incubation with a protease the IL-6i activity disappears.

Accordingly, in one aspect, the invention is directed to a composition of matter having IL-6 inhibitory activity, said composition comprising a cell culture supernatant wherein the cells have been stimulated for production of IL-6 inhibitory activity, partially purified forms of the IL-6i components of the supernatants or the individual inhibitory components thereof.

The invention includes also functional derivatives of said proteins which may be obtained, using well known methods, by deleting, adding or substituting residues of amino acids in said sequence while the functional IL-6i activity is retained. The change of amino acids may involve only one residue or various residues, in particular 1-50 amino acids residues.

In another aspect, the invention is directed to pharmaceutical compositions containing a partially purified cell culture supernatant having IL-6i activity from mammalian cells stimulated for IL-6i production, or a component thereof having IL-6i activity, to methods to treat inflammation using these compositions, and to methods to prepare the compositions of the invention.

## Brief Description of the Drawings

General: in the described Figures the Y-axis often gives the IL-6 activity. A low value in relation to a IL-6 standard or to a reference sample points to a corresponding high IL-6i content of the assay sample.

Figure 1 shows the IL-6 inhibiting activity of fractions obtained by HPLC gel filtration of an IL-6i-containing supernatant, prepared with human PBMCs under UV stimulation as described in Example 1.

Figure 2 shows the IL-6 inhibiting activity of five IL-6i-containing supernatants after culture stimulation with different amounts of UV light.

Figure 3 shows the development of IL-6 inhibiting activity in a culture of PBMC cells with irradiation (hatched bars) and without irradiation (blank bars).

Figure 4 shows the IL-6 inhibiting activity of fractions obtained by HPLC gel filtration of IL-6i-containing supernatant prepared with KB cells under PMA stimulation as set forth in Example 2.

Figure 5 shows the IL-6 inhibiting activity of a supernatant prepared according to Example 3 with KRFM cells under PMA stimulation (crossed bar) and without stimulation (hatched bar). Native IL-6 has not been removed from the assay samples.

Figure 6 shows the IL-6 inhibiting activity of fractions obtained by anion exchange chromatography of a supernatant prepared according to Example 5 with A431 cells under IL-1/IL-6 stimulation. Native IL-6 has not been removed from the assay samples.

Figure 7 shows the optical density (280 nm) of fractions eluted from a HIC column onto which was applied an ammonium sulfate treated supernatant prepared according to Example 6 with A431 cells under PMA stimulation.

Figure 8 and Figure 9 show the IL-6 inhibiting activity and the IL-1 inhibiting activity respectively of increasingly diluted fractions eluted from a HIC column as described for Figure 7.

Figure 10 shows the IL-6 inhibiting activity of precipitates (middle bars) obtained by successive addition of ammonium sulfate until 60% and 75% saturation respectively, to crude supernatant obtained from the culture of IL-6i producing cells, compared with the activities of crude supernatant (most left bar) and remaining supernatant (most right bar).

Figure 11 shows the IL-6 inhibiting activity of fractions obtained by anion exchange chromatography of fraction 3 shown in Figure 7. Native IL-6 has not been removed from the assay samples.

Figure 12 shows the IL-6 inhibiting activity of fractions obtained by HPLC gel filtration of the pooled IL-6i fractions 4 and 5 of the anion exchange chromatography illustrated by Figure 11. Molecular weight markers for 80 kDa and 8 kDa are indicated.

Figure 13 shows the inhibitory activity of a purified supernatant when assayed in a IL-1, IL-2, IL-4 and IL-6 test as described in Example 8.

Figure 14 shows the IL-6i activity of increasingly diluted, prostate epithelial cell culture derived supernatants, prepared with PMA (right bar) and without PMA (left bar) as described in Example 7.

Figure 15 shows the IL-6 inhibiting activity of fractions obtained by anion exchange chromatography of a supernatant prepared according to Example 4 with A431 cells under PMA stimulation, after ammonium sulfate (75%) precipitation. Native IL-6 has not been removed from the assay samples.

Figure 16 shows the IL-6 inhibiting activity of fractions obtained by reversed phase chromatography of the IL-6i activity containing fractions of the separation as described in Figure 15.

Figure 17 shows the IL-6 inhibiting activity of fractions obtained by gel permeation chromatography of the IL-6i activity containing fractions of the separation as described in Figure 16.

<u>Modes of Carrying Out the Invention</u>

Abbreviations used herein include:

| | |
|---|---|
| PBS | = phosphate buffered saline; |
| PMA | = phorbol myristate acetate; |
| KRFM | = indication of a human nodular melanoma cell line; |

RPMI 1640 is a standard medium (JAMA (1967) 199:519)

| | |
|---|---|
| $J/m^2$ | = $Joules/meter^2$; |
| FCS | = fetal calf serum; |
| EDTA | = ethylenediaminotetraacetic acid; |
| PBMC | = peripheral blood mononuclear cells; |
| HEPES | = [4-(2-hydroxyethyl)-1-piperazine-ethanesulfonic acid] |
| IL | = interleukin |
| hIL-1 | = human IL-1; |
| hIL-6 | = human IL-6; |
| IL-6i | = IL-6 inhibitor or antagonist; |
| rhIL-6 | = recombinant human IL-6. |
| FPLC | = fast protein liquid chromatography |

The IL-6 inhibiting compositions of the invention are prepared by the stimulation of human or animal cells, particularly mammalian blood cells or cell lines, and optional further purification from the culture supernatants. Among cells suited for carrying out the invention are human adherent peripheral blood mononuclear cells (PBMC), human epidermoid carcinoma cells, melanoma cells and prostate epithelial cells, but other human or animal cells may be used as well. For the stimulation of these cells can be used, for example, ultraviolet (UV) radiation or phorbol myristate acetate (PMA) or a mixture of IL-1 and IL-6 or a combination of these stimulants. Other suitable stimulants can be found in the group comprising stimulants of protein kinase C and and enhancers of the intracellular calcium concentration. Suitable amounts of UV radiation are 100 - 150 J/$m^2$. Suitable doses of PMA are 10 - 100 ng/ml.

After stimulation the cells are grown during 24-48 hours, preferably 36 hours under standard culture conditions. Then the supernatants are harvested.

Preceding or following further purification an assay for determining IL-6i activity is carried out. This assay is based on the reduction of IL-6 stimulated growth of IL-6 dependent cells.

In one manner of conducting the assay, samples to be tested are freed from any native IL-6. A predetermined amount of IL-6 is then added to the samples supposed to contain IL-6i and after admixture with a IL-6 dependent cell line cell growth is determined.

According to a typical and workable protocol supernatant is first freed from native IL-6. A suitable procedure is addition of ammonium sulfate until 75% saturation. As can be seen in Figure 10 IL-6 is in the precipitate at 60% saturation, while the IL-6i is still present in the supernatant after 75% saturation. Alternatively IL-6 can be removed by passing a sample through an affinity chromatography column bearing antibodies to IL-6. The columns for affinity chromatography are prepared by crosslinking the IgG fraction of a rabbit anti-hIL-6 antiserum to cyanogen bromide-activated Sepharose 4B (Sigma Chemical Corp., St. Louis, MO).

A suitable IL-6 dependent hybridoma cell line is B9, which can be obtained from L. Aarden, University of Amsterdam, The Netherlands. Other suitable IL-6 dependent lines are generally available or can be prepared according to methods well known in the art. For instance with the method described in "A growth-factor dependent B-cell hybridoma", Lansdorp, P.M. et al., Current Topics in Microbiology and Immunology (1986) 132:105-113. A subclone obtained from the described IL-6 dependent B13.29 cell line is denoted "B9".

Serial dilutions of the test samples (preferably freed from native IL-6) are applied in triplicate on 96-well flat bottom microtiter plates.

5 x $10^3$ B9 cells in 50 $\mu$l medium are added to each well.

An IL-6 activity baseline is created by adding to each well IL-6 solution until a a fixed calculated concentration in the range of 1-10 U/ml, preferably 1 U/ml. A suitable form of IL-6 to be added is rec-DNA prepared human IL-6 (rhIL-6) and marketed by, e.g., British Biotechnology.

After a 72 hrs incubation period a 4 h pulse of 0.1 $\mu$Ci [$^3$H]-thymidine is added. The cells are then harvested and washed using a cell harvester. The amount of [$^3$H]-thymidine in the cells is determined in a liquid scintillation counter; the incorporation of [$^3$H]-thymidine in the cells depends on the IL-6 activity. Test wells activities are compared to the baseline activity. Results are expressed as counts per minute (CPM) or as U/ml, by comparison with a relevant IL-6 standard curve. Means and Standard Deviations (SD) are calculated for assays in triplicate.

Supernatants having IL-6i activity can be subjected to purification techniques suitable for isolation of individual IL-6i components or for partial purification thereof. Suitable techniques are e.g. anion exchange chromatography, HPLC size exclusion chromatography and hydrophobic interaction chromatography. These techniques can suitably be used separately, but more more advantageously in combination, particularly successively.

According to one successful separation protocol, the supernatants obtained from a stimulated cell culture are first concentrated by ultrafiltration and subsequently admixed with ammonium sulfate until 75% (w/v) saturation. The IL-6i activity remains in the supernatant (Figure 10). In order to remove ammonium sulfate the supernatant is dialysed (against 10 mM TRIS/HCl, pH 7.9) and applied onto a column for anion exchange chromatography. The column may preferably be a DEAE monoQ column (e.g. the 16/100 column of Pharmacia). Fractions are eluted at pH 7.9 and a flow rate of 4 ml/min with a 10 mM Tris-Cl buffer, containing a linear NaCl gradient. Collected fhe fractions are assayed for IL-6i as set forth above. A fraction containing IL-6 inhibitory activity can be detected at 30-100 mM NaCl. This fraction contains a component, which according to size exclusion chromatography has a Mw of about 10 kDa.

After desalting, the selected fractions are concentrated by ultrafiltration to obtain components having IL-6 inhibitory properties useful for pharmaceutical compositions.

Another suitable separation method is based on hydrophobic interaction chromatography (HIC). A suitable column for HIC is the TSK Butyl 650 column (E. MERCK). A sample is admixed with ammonium sulfate until a final concentration of 1 M and applied upon the HIC column. For elution a step gradient is used comprising ammonium sulfate in the concentrations 1 M, 0.5 M, 0.1 M and 0 M and, finally, ethylene glycol/water (80% v/v).

A further separation method which can be used for purifying crude IL-6i containing samples is reversed phase chromatography (RP chromatography). A suitable column is the BIORAD HI-pore RP304 column with water as the mobile phase. For elution an acetonitrile gradient may be used ranging from 0 to 100% acetonitrile at a flow rate of 1.2 ml/min.

Supernatants or the active fractions from anion exchange chromatography may also be purified using gel filtration (= gel permeation) HPLC on the basis of molecular weight. The HPLC system does not have specific requirements. A suitable system is supplied by LKB comprising two LC 2150 pumps, a system controller model 2152, and a Rheodyne 7010 injector, coupled to a Uvicon 720 LC variable wavelength detector (Kontron, Vösendorf, Austria).

Molecular weights can be established by assessment in a gel filtration size exclusion column, using standard molecular weight markers.

A typical procedure for obtaining the IL-6i factors of the invention as applied in the Examples below comprises the following steps:

Cells suited for the production of IL-6 inhibitors ($1x10^6$ cells/ml) are washed in PBS.

For UV stimulation cells are treated with a single single UV exposure of 100 - 150 J/$m^2$. The cells ($1x10^6$) are washed twice in RPMI 1640 (Flow Laboratories, McLean, VA), incubated in serum-free RPMI 1640 at 37°C in a humidified atmosphrere containing 5% $CO_2$. Supernatants are harvested 24 hr after irradiation and, optionally, stored at -20°C.

For PMA stimulation, cells are grown as indicated for the cell line employed. After they reach confluency, the medium is changed and PMA (10 - 100 ng/ml, preferably 50 ng/ml) (Sigma Chem. Corp., St. Louis, MO) is added. Supernatants are harvested 24 hr after the addition of PMA and, optionally, stored at -20°C.

For purification of the IL-6i factor using size separation, 100 µl samples of the supernatants are subjected to size-exclusion chromatography using HPLC as set forth with particularity in the Examples below and the obtained fractions are, optionally, stored at -20°C.

Alternatively, separation is carried out using DEAE ion exchange chromatography or hydrophobic interaction chromatography. Fractions are concentrated by ultrafiltration and, optionally, desalted, followed by the IL-6i assay of the samples as described above. IL-6i containing fractions are collected and, optionally, lyophilized. Two hundred liters of culture liquid yield approximately 50 µg IL-6 inhibitor. Fractions were desalted by dialysis against PBS and tested in the B9-assay.

One embodiment of this invention is a factor with IL-6i activity and a MW of approximately 10 kDa. This factor is further characterized in that it does not inhibit either IL-1, IL-2 or IL-4 (see Figures 9 and 13).

Another IL-6i embodiment is a factor of approximately 44 kDa.

The products of the invention may also be obtained by recombinant DNA techniques according to methods known to the man skilled in the art. See for instance: Maniatis et al., Molecular cloning: A Laboratory Manual, Cold Spring Harbor Laboratory, CSH, NY (1982). The IL-6 inhibitor is obtained by culturing transformed host cells containing the DNA encoding the gene for the inhibitor in an expressible form. To achieve this the IL-6i encoding DNA must be cloned in a suitable expression system. The DNA is selected using appropriate probes for hybridisation. Transformed host cells which secrete IL-6i factor are identified by labeled radioactive antibodies which have been raised against the IL-6i proteins.

Particularly useful recombinant host organisms for large scale production of a IL-6i protein are Bacillus licheniformis, Kluyveromyces lactis and Aspergillus niger.

The invention also comprises pharmaceutical preparations containing the IL-6i compositions or individual components thereof. The crude supernatants, herein-described, partially purified IL-6i components or pure IL-6i components can be used. If prepared as the cell culture supernatant or from the cell culture supernatants, the IL-6i compositions are initially isolated as aqueous solutions of the active principle. After desalting and lyophilization a stable powder results in which the IL-6 inhibiting activity is preserved. The crude supernatant or partially purified composition may be processed further to remove impurities which are associated with the cell culture or which result from the production process. The IL-6i compositions either as solutions or as powders, in crude or in purified form, may be processed by methods known in the pharmaceutical art to pharmaceutical preparations which are beneficial for the treatment of IL-6-mediated disorders, such as psoriasis, allergic reactions, rheumatic afflictions and inflammatory diseases of the skin, the lung or the gastrointestinal tract and for treating burns and ulcers.

Particularly preferred are preparations which are suited for intravenous, subcutaneous, intralesional, oral or topical administration, such as injection fluids, tablets, creams, ointments or lotios. Other dosage forms can also be used. Techniques for formulation of the IL-6i compositions are available in the art, and can be found, for example, in Remington's Pharmaceutical Sciences (latest edition), Mack Publishing Co., Easton, PA. Satisfactory results may be obtained with a pharmaceutical preparation made with the crude isolate, but it is preferred to use purified IL-6 inhibitor (IL-6i).

The following Examples are intended to illustrate but not to limit the invention.

**Preparation A**

Isolation of Human Peripheral Blood Mononuclear Cells

About 15 to 20 ml of Lymphocyte Separation Medium (Flow) was overlaid with 30 ml of human blood, and the mixture was centrifuged for 20 min at 1800 rpm. The interface (which contains white blood cells) was carefully removed and washed with Hank's Balanced Salt Solution. The cell pellet was resuspended in RPMI 1640 with 5 to 10% (v/v) FCS, the cell density was adjusted to 5 x $10^6$ cells/ml. Ten ml cell suspension was incubated in a 9 cm plastic Petri dish for at least 2 hours at 37° C to allow the PBMC's to adhere. The supernatant was then removed and the cells were washed carefully with RPMI. Attached cells could be harvested using a rubber policeman.

**Example 1**

Preparation of IL-6i Using Peripheral Blood Mononuclear Cells

Human PBMC's (adherent cells) prepared as described in Preparation A were maintained and grown in monolayer culture using RPMI 1640 supplemented with 5% (v/v) FCS. In order to subculture the cells, they were first washed with PBS (pH 7.4) and then treated at 37° C with 0.05% (w/v) trypsin and 0.02% (w/v) EDTA in PBS. Three-milliliter aliquots of cell suspension (1x$10^6$ cells/ml) were placed in 60 mm plastic petri dishes followed by a single UV exposure of 100 J/$m^2$. Osram Vitalux bulbs emitting a continuous spectrum between 300 and 600 nm were used. The output was 0.2 mJ/$cm^2$/sec at 297 nm as measured with an International Light 700 Research Radiometer.

Subsequently the cells were washed twice in RPMI 1640 (Flow Laboratories, McLean, VA), resuspended in 1 ml of serum-free RPMI 1640, and incubated at 37° C in a humidified atmosphere with 5% $CO_2$. Supernatants were harvested 24 hr after irradiation and lyophilized. Samples (100 $\mu$l) were subjected to HPLC gel filtration using a TSK-25 column (E. Merck). For this separation (based on size of the products contained in the supernatants) the size-exclusion column, Bio-Sil TSK 125, 300 x 7.5 mm of BIO-RAD was used. Elution was carried out with PBS, pH 7.2, at a flow rate of 1.0 ml/min. Column fractions (0.5 ml) were diluted with RPMI 1640 containing 5% FCS, then filter sterilized. Columns were calibrated with a gel filtration standard (BIO-RAD, Richmond, CA) containing bovine thyroglobulin (670 kDa), bovine gamma-globulin (158 kDa), chicken ovalbumin (44 kDa), horse myoglobulin (17 kDa) and cyanocobalamin (1.3 kDa).

The fractions were tested for IL-6i activitiy using the assay described above after admixture with a 1 U/ml rhIL-6 standard sample. These activities are shown in Figure 1.

The X-axis of the figure shows the column fractions and the Y-axis the IL-6 activity (expressed in counts per minute). The horizontal line shows the activity of an IL-6 (1 U/ml) standard sample before its admixture with the sample to be assayed. The molecular weight markers are shown at the top of the figure. The minima in the curve for the fractions 2-6 and 11-13 respectively point to the presence of IL-6 inhibitors of approximately 10 kDa and 44 kDa.

To determine the effect of varying amounts of UV stimulation five IL-6i containing supernatants were prepared as described above but with amounts of UV-energy increasing from 0-150 J/$m^2$. These supernatants were assayed on IL-6i activity, but without previous removal of native IL-6, neither was a standard amount of IL-6 added to the assay sample. The supernatants were diluted 1:300. See Figure 2. The Y-axis of the figure shows the IL-6 activities (expressed in counts per minute times 1000). The number under each bar means the total amount of UV-light administered to the corresponding sample expressed in J/$m^2$. As shown, increasing amounts of light initially enhanced the production of IL-6 activity, putatively by induction of greater production of IL-6. However, with an influx of light increasing over 50 J/$m^2$ the production of IL-6 inhibitor or inhibitors superseded the IL-6 increase, resulting in a diminishing net IL-6 activity in the supernatant of highly irradiated cells. The bars are provided with the standard deviation indications.

Figure 3 shows the kinetics of IL-6 and IL-6 inhibitor production in this system. Human PBMC cells were treated and cultured as above. Samples were taken at 0, 2, 4, 8, 16 and 32 hours. Neither was native IL-6 removed, nor was a standard amount of IL-6 added before supernatants were assayed for IL-6 inhibiting activity. The IL-6 activities of cells irradiated with 100 J/$m^2$ UV light are indicated by the hatched bars corresponding to the sampling times; the open bars represent reference supernatants of cells not irradiated taken at the same times. IL-6 activities are indicated in counts per minute (times 1000); standard deviations are shown. When compared to the not-stimulated samples (open bars), the UV treated PBMC supernatants (hatched bars) demonstrate significantly less IL-6 activity. Since UV treated PBMC's produce IL-6 activity, it is concluded that this indigenous IL-6 was neutralized by the IL-6 inhibitor produced concomitantly.

**Example 2**

Preparation of IL-6i Using KB Cells

Human epidermoid carcinoma KB cells (ATCC CCL17) are obtainable from the American Type Culture Collection. These cells were cultured and stimulated with PMA (10 ng/ml) and the supernatants were lyophilized and subjected to HPLC gel filtration (TSK 125), as described in Example 1. The fractions were tested in the B9 assay in the presence of 10 U/ml rhIL-6 as described above. Their activities are shown in Figure 4. The X-axis of the figure shows the fractions and the Y-axis the IL-6 activities (expressed in counts per minute times 1000). The horizontal line shows the activity of an IL-6 (10 U/ml) standard sample before its admixture with the sample to be assayed. The molecular weight markers are shown at the top. The minima in the curve for the fractions 3, 5-7 and 15-19 point to the presence of IL-6 inhibitors, again having approximate molecular weights of 10 kDa and 44 kDa.

**Example 3**

Preparation of IL-6i Using KRFM Cells

Human melanoma KRFM cells obtained from professor Th. Luger, University of Münster, Germany, were grown and after they had reached confluency, the medium was substituted by a medium without FCS. The cells were incubated for 24 hours with PMA (10 ng/ml) and without PMA. The supernatants were recovered and subjected to IL-6 affinity chromatography, as described above, so that no IL-6 activity remains in the eluate. The IL-6i activity of the eluate was then assessed after admixture with a 10 U/ml rhIL-6 standard sample and the results are shown in Figure 5.

The bars indicate the IL-6 activities in counts per minute times 1000 and the standard deviations. The open bar shows the IL-6 activity of a rhIL-6 standard sample (10 U/ml). Supernatant prepared with PMA stimulation (crossed bar) shows far less IL-6 activity, apparently because a strong IL-6 inhibiting substance is produced. Supernatants prepared without PMA stimulation (hatched bar) the eluate did not show significant inhibition of IL-6 activity as compared with the standard sample.

**Example 4**

Preparation of IL-6i Using A431 Cells

Human epidermoid carcinoma A431 cells (ATCC CRL1555) are a fast growing cell line obtainable from the American Type Culture Collection. The cells were cultured in standard culture medium. At confluency the culture medium was removed and substituted with stimulation medium, which comprised: DMEM/F12 (1:1), 20 mM glutamine, antibiotics (e.g. 100 U/ml penicillin, 100 μg/ml streptomycin and 0.5 μg/ml fungizone), 10 mM HEPES, and as stimulants 200 nM ionomycine and 50 ng/ml PMA.

After 36 hrs of stimulation the supernatant was harvested. A protease inhibitor, such as 10 mM PMSF (phenyl methyl sulfonyl fluoride), was added and the conditioned medium was centrifuged at 2000 g for 15 to 20 min.

The clarified supernatant was concentrated immediately, using an ultrafiltration unit with a cut off of 3 kDa. Concentration by ultrafiltration could suitably be carried out using an Amicon stirred cell equiped with a YM5 filter. Purification of the retentate using ammonium sulfate precipitation (see fig. 10), anion exchange chromatography, gel filtration chromatography and assay of the fractions using the B9 bioassay were carried out as described above.

**Example 5**

Preparation of IL-6i Using A431 Cells

Human epidermoid carcinoma cells (A431) were incubated with a mixture of hIL-1 (1 U/ml) and rhIL-6 (100 U/ml) for 36 hours. Supernatants were dialyzed against water. The dialysates were freeze dried on behalf of further concentration and subjected to DEAE ion exchange chromatography as described above. The fractions were dialyzed against water, reconstituted using 10 x RPMI 1640 and subsequently tested in the B9 bioassay for IL-6i described above. Activities of the column fractions are shown in Figure 6.

The X-axis of the figure shows the fraction numbers and the Y-axis the IL-6 activities expressed in counts per minute. The dotted line refers to the NaCl gradient ranging from 0 to 500 mM NaCl. The dashed line represents the activity of an IL-6 (1 U/ml) standard sample before its admixture with the sample to be assayed. The two minima in the solid line, corresponding to fractions 6-10 and 22-24, respectively, refer to the IL-6 inhibiting substances.

**Example 6**

Preparation of IL-6i Using A431 Cells

Human epidermoid carcinoma cells (A431) were cultured and stimulated as described in Example 4. The supernatant was concentrated and dialysed (against 10mM TRIS/HCl, pH 7.0). Ammonium sulfate was added until a final concentration of 1 M. After centrifugation of the suspension the clear supernatant was applied onto a column for hydrophobic interaction chromatography (TSK Butyl 650, E. MERCK). Elution was carried out using first a stepwise ammoniumsulfate gradient, followed by TRIS/HCl and ethylene glycol:

fraction 1 : 1.0 M ammonium sulfate
fraction 2 : 0.5 M ammonium sulfate
fraction 3 : 0.1 M ammonium sulfate
fraction 4 : 10 mM TRIS/HCl (pH 7.8)
fraction 5 : ethylene glycol/water (80/20, v/v).

The results are shown in Figure 7 where the left Y-axis gives the optical density at 280 nm of the fractions and the right Y-axis the ammonium sulfate concentration in M.

After dialysis against RPMI 1640 IL-6i activity was assayed using the B9 assay described above (Figure 8). Each of the fractions 1-5 is represented by a line. The Y-axis gives the IL-6 activity in counts per minute. The horizontal reference line is the activity of an IL-6 (1 U/ml) standard sample before its admixture with the sample to be assayed. Fraction 3 clearly contained most IL-6i activity, followed by fraction 4. The assay has been carried out for four different dilutions of the five fractions.

The same fractions were also assayed (see Example 8) for inhibition of IL-1 activity, as expressed as proliferation inhibition of IL-1 dependent thymocyte growth (Figure 9) and for inhibition of IL-2 and IL-4 activity (see Figure 13). In Figure 9 the Y-axis gives the IL-1 activity in counts per minute. The right Y-axis gives the NaCl concentration. The X-axis gives the fraction numbers. The horizontal reference line is the theoretical activity of an added IL-1 standard sample containing 1 U/ml. It is clear that none of the fractions showed any inhibiting activity on the added IL-1 standard substance. The assay was carried out for six different dilutions of the five fractions. No inhibition of IL-1 activity appeared to be present.

Fraction 3 was dialysed against TRIS/HCl (10mM, pH 7.8) and applied onto an anion exchange column for FPLC chromatography. Elution was carried out using TRIS/HCl (10 mM, ph 7.9), containing a linear sodium chloride gradient (0-500 mM). The IL-6i factor eluted in the fractions containing 30-60 mM sodium chloride (Figure 11). The left Y-axis gives the IL-6 activity in counts per minute. The horizontal reference line is the activity of an IL-6 (1 U/ml) standard sample before its admixture with the sample to be assayed.

The fractions from anion exchange showing IL-6i activity were pooled, concentrated by dialysis against polyethylene glycol 8000 and applied onto a 60 cm TSK 2000 column (LKB) for HPLC gel filtration chromatography using PBS as eluens. The IL-6i activity was associated with the fractions corresponding to about 10 kDa and 80 kDa respectively (Figure 12) according to molecular weight markers A and B. The Y-axis gives the IL-6 activity in counts per minute. The horizontal reference line is the activity of an IL-6 (1 U/ml) standard sample before its admixture with the sample to be assayed. That IL-6i activity was found not only at about 10 kDa, but also at 80 kDa. This phenomenon can possibly be explained by formation of an aggregate of the lighter IL-6i factor.

**Example 7**

Preparation of IL-6i Using Prostate Epithelial Cells

Prostate epithelial cells PC3 are obtainable from the American Type Culture Collection (ATCC CRL 1435). The cells were cultured and stimulated and the supernatants were recovered and tested as described in Example 4 for A431 cells.

Figure 14 shows the IL-6i activity of these supernatants, prepared with PMA (right bar) and without PMA (left bar). Four pairs of bars represent four dilutions (1:4, 1:8, 1:16, 1:32) of the supernatants. PMA stimulated cells evidently produce IL-6i.

**Example 8**

Assay on inhibition of IL-1, IL-2 and IL-4

General

Test samples were 1:1 diluted with supplemented RPMI 1640. Alternatively specimens can be dialysed against water and reconstituted with 10xRPMI 1640. After filter sterilisation samples optionally can be stored at -20° C.

Inhibition of interleukin 1

Thymocytes of of 6 - 8 weeks old C3H HeJ mice (available from IFA CREDO) were suspended in 15 ml RPMI 1640, supplemented with 5% heat inactivated FCS, 20 mM glutamine, 100 U/ml penicillin, 100 μg/ml streptomycin, 10 mM HEPES, 10 mM sodium pyruvate and 0.05 mM 2-mercaptoethanol, until only connective tissue remained. Thymocyte suspension was filtered by using a conically bent steel grid into a 15 ml falcon tube and centrifuged at 1200 rpm (36 cm rotor diameter) for 8 minutes. Washing was repeated two times and the thymocyte concentration adjusted to $3.10^7$/ml.

A standard curve is made, using several dilutions, and using an IL-1 standard (3 U/ml, obtainable from FLOW) prepared as described above. Control samples were included. To all wells (except the control wells) 50 μl of a concanavalin A solution (2.2 μg/ml, obtainable from PHARMACIA) was added. To all wells natural human IL-1 was added until 0.05 U/well. Then 50 μl of the above described thymocytes suspension was added to each well. After 67 hours 0.1 μCi tritiated thymidine in 50 μl RPMI 1640 medium was added. Incorporated radioactivity was determined in a liquid scintillation counter. Results were expressed as the mean of triplicate experiments.

IL-2 and IL-4 inhibition

A suspension in 100 μl of 1*$10^4$ CTLL cells, obtainable from the American Type Culture Collection (ATCC TIB214), were cultured in RPMI 1640 medium with 5% FCS, 20 mM glutamin, 100 U/ml penicillin, 100 μg/ml streptomycin, 10 mM HEPES, 10 mM sodium pyruvate and 0.05 mM 2-mercaptoethanol. To each well 100 μl test sample was added. After 2 days the proliferation of the CTLL cells was determined using $^3$H-thymidine incorporation measurement as described for the IL-1 assay.

For determining IL-2 inhibiting activity 0.05 U human IL-2 was added to each well.

For determining IL-4 activity 0.05 U recombinant mouse IL-4 was added to each well.

Figure 13 shows the inhibitory activity of a purified supernatant when assayed in a IL-6, IL-2, IL-4 and IL-1 test. Only exogeneous IL-6 activity was reduced by the samples. Exogeneous IL-1, IL-2 and IL-4 activities were not affected.

**Example 9**

Purification of an IL-6i containing supernatant

Human epidermoid carcinoma cells (A431) were cultured and stimulated as described in Example 4. The supernatant was concentrated and dialysed (against 10 mM TRIS/HCl, pH 8.9).

This material was applied upon a MonoQ AEX column and eluted using a linear NaCl gradient ranging from 0 to 100 mM during the first 30 minutes. The NaCl concentration was increased during min 33 to min 38 until reaching a final concentration of 1000 mM, kep at that concentrtaion for 10 min and finally decreased to 0 mM during the last 5 min. The IL-6 inhibiting activity eluted as a single peak at a NaCl concentration of 80 - 90 mM (Figure 15).

Active fractions 30 and 31 were pooled and further separated by reversed phase (RP18) chromatography, using water as mobile phase. A linear gradient ranging from 0 to 100% of acetonitrile was used. The IL-6 inhibitor eluted as a single peak of activity within an acetonitrile range of 50 to 60% (Figure 16).

The RP18 fractions 22, 23 and 24, containing about 150 μg IL-6i, were pooled and further separated by gel permeation chromatography (GPC). The IL-6i inhibitory activity eluted in two distinct peaks of inhibitory activity demonstrating activity at molecular weights of about 44 and 10 kDa (Figure 17).

## Claims

1. A composition having interleukin-6 inhibiting activity (IL-6i), wherein said composition is a partially purified cell culture supernatant having IL-6i activity from mammalian cells stimulated for IL-6i production, or a component thereof having IL-6i activity.

2. The composition of claim 1, wherein said IL-6i activity is associated with a protein, which
   - has a molecular weight, assessed in a gel filtration size exclusion column, of approximately 10 kDa, and
   - has no interleukin-1, interleukin-2 or interleukin-4 inhibiting activity, and
   - does not precipitate in a 75% saturated ammonium sulfate solution.

3. The composition of claim 1, wherein said IL-6i activity is associated with a protein having a molecular weight, assessed in a gel filtration size exclusion column, of approximately 44 kDa.

4. A purified IL-6i factor obtainable by a process comprising:

   treating a culture of human adherent PBMC cells, epidermoid carcinoma cells, melanoma cells or prostate epithelial cells with UV light and/or with phorbol myristate acetate and/or with an IL-1 and IL-6 mixture,

   separating the cell culture supernatant into fractions,

   assaying the fractions for IL-6i activity;

   recovering at least one fraction with IL-6i activity, and

   optionally concentrating and/or further purifying said fraction.

5. The purified IL-6i factor of claim 4 wherein said separating is by ion exchange, hydrophobic interaction chromatography or reversed phase chromatography.

6. The purified IL-6i factor of claim 4 wherein said separating is based on molecular size.

7. The factor of claim 4 which has a MW of approximately 10 kDa.

8. The factor of claim 4 which has a molecular weight of approximately 44 kDa.

**9.** A factor in a purified and isolated form having interleukin-6 inhibiting activity, being a protein and characterized by a molecular weight, assessed in a gel filtration size exclusion column, of approximately 10 kDa and by the absence of IL-1, IL-2 or IL-4 inhibiting activity.

**10.** A factor in a purified and isolated form having interleukin-6 inhibiting activity, being a protein and characterized by a molecular weight, assessed in a gel filtration size exclusion column, of approximately 44 kDa.

**11.** A process for the preparation of an interleukin-6 inhibiting factor comprising the following steps:

treating a culture of human adherent PBMC cells, epidermoid carcinoma cells, melanoma cells or prostate epithelial cells with UV light and/or with phorbol myristate acetate and/or with a IL-1 and IL-6 mixture,

recovering the cell culture supernatant,

separating the components of the supernatant into fractions.

**12.** The method of claim 11 wherein said separating is by ion exchange or hydrophobic interaction chromatography.

**13.** The method of claim 11 wherein said separating is based on molecular size.

**14.** A pharmaceutical formulation for controlling or ameliorating psoriasis, allergic reactions, rheumatic afflictions and inflammatory diseases of the skin and the lungs comprising an effective amount of a composition having interleukin-6 inhibiting (IL-6i) activity and one or more pharmaceutically acceptable excipients, diluents and/or adjuvants.

**15.** The formulation of claim 14 wherein the IL-6i composition is a protein having a molecular weight, assessed in a gel filtration size exclusion column, of approximately 10 kDa.

**16.** The formulation of claim 14 wherein the IL-6i composition is a protein having a molecular weight, assessed in a gel filtration size exclusion column, of approximately 44 kDa.

**17.** Treatment of an inflammatory disease characterized in that an effective amount of an IL-6i factor containing pharmaceutical composition is administered.

Figure 1

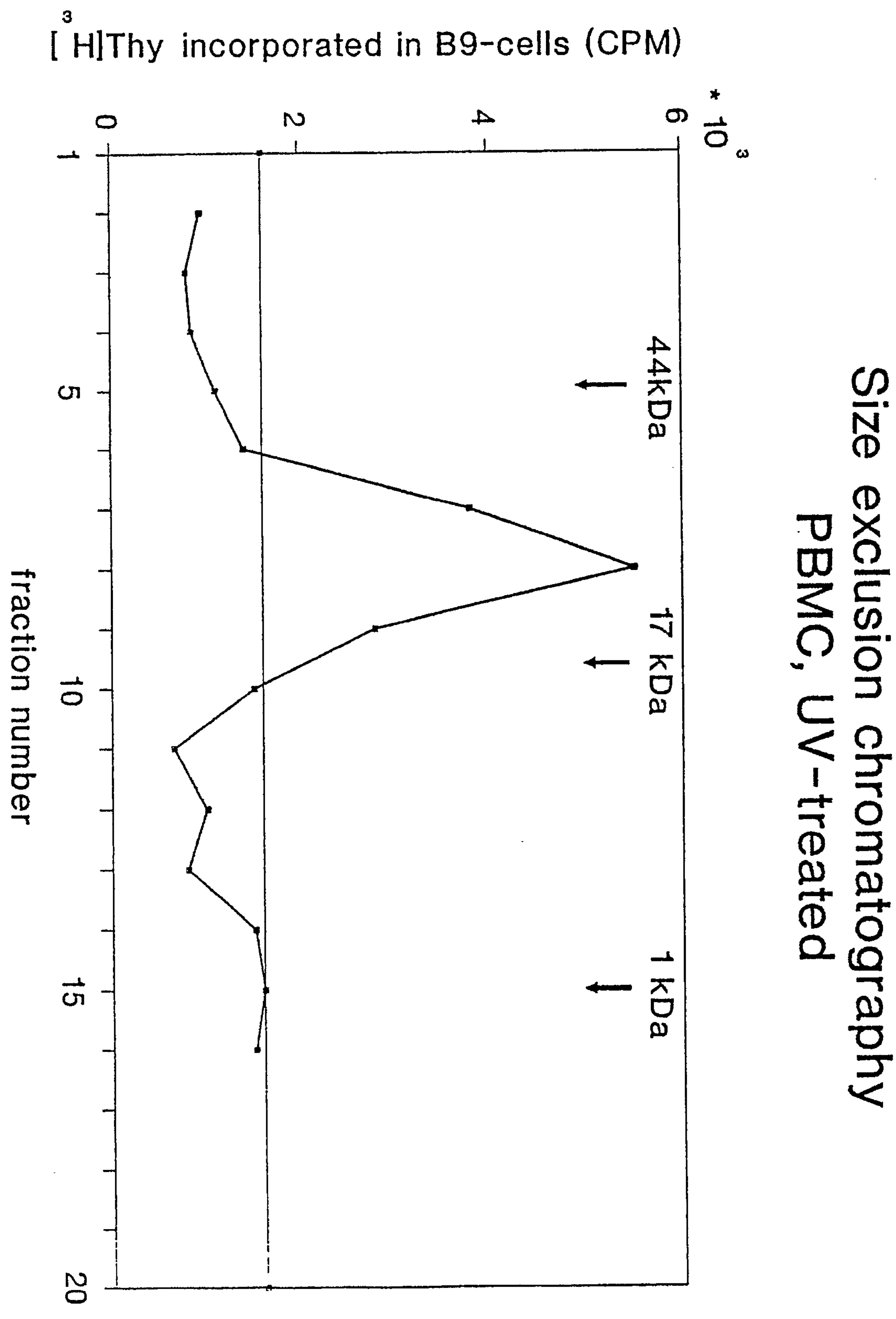
Size exclusion chromatography
PBMC, UV-treated
[3H]Thy incorporated in B9-cells (CPM)
* 10 3
0
2
4
6
44kDa
17 kDa
1 kDa
1
5
10
15
20
fraction number

Figure 2

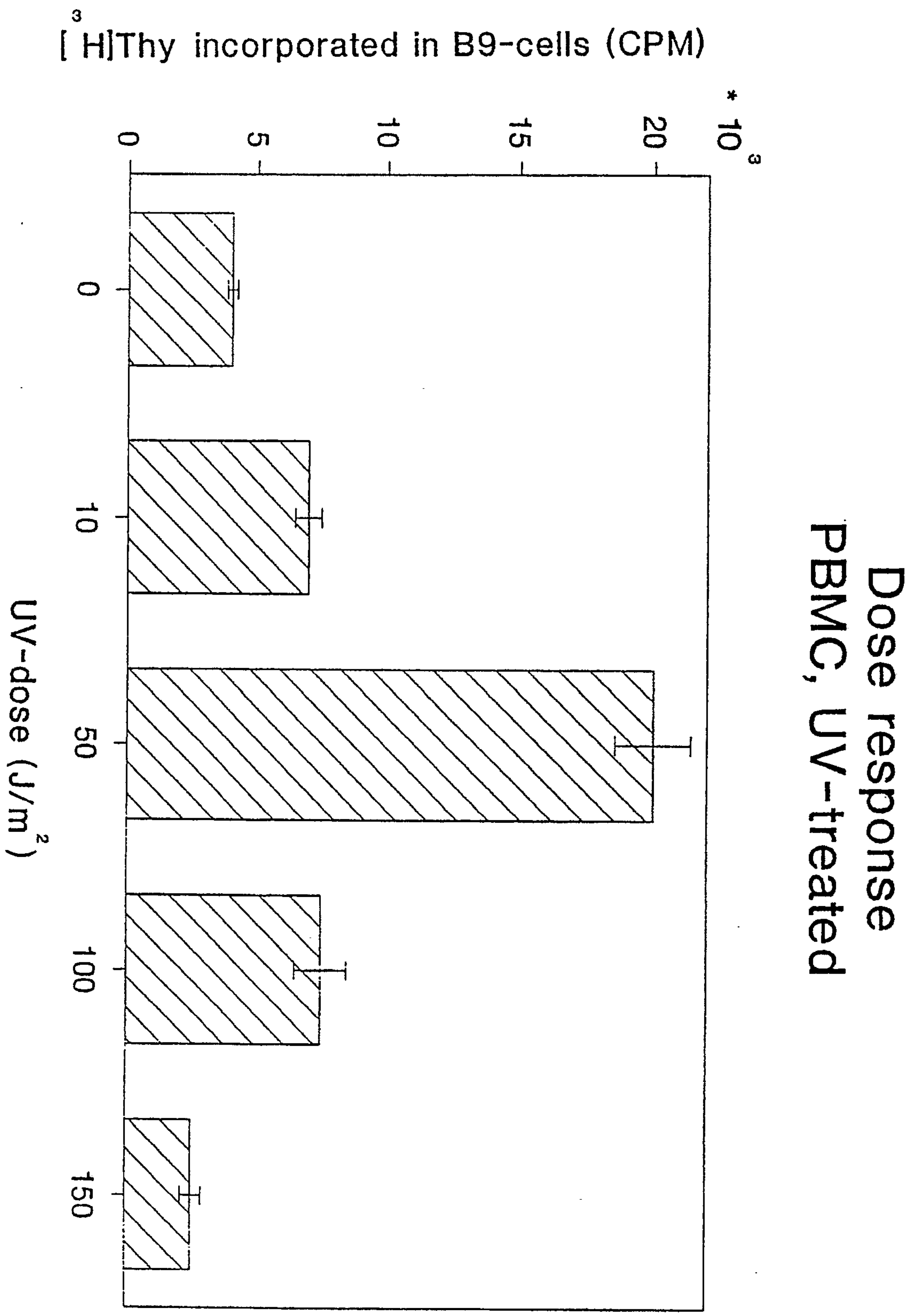
Dose response
PBMC, UV-treated
[3H]Thy incorporated in B9-cells (CPM)
* 10 3
0
5
10
15
20
0
10
50
100
150
UV-dose (J/m 2 )

Figure 3

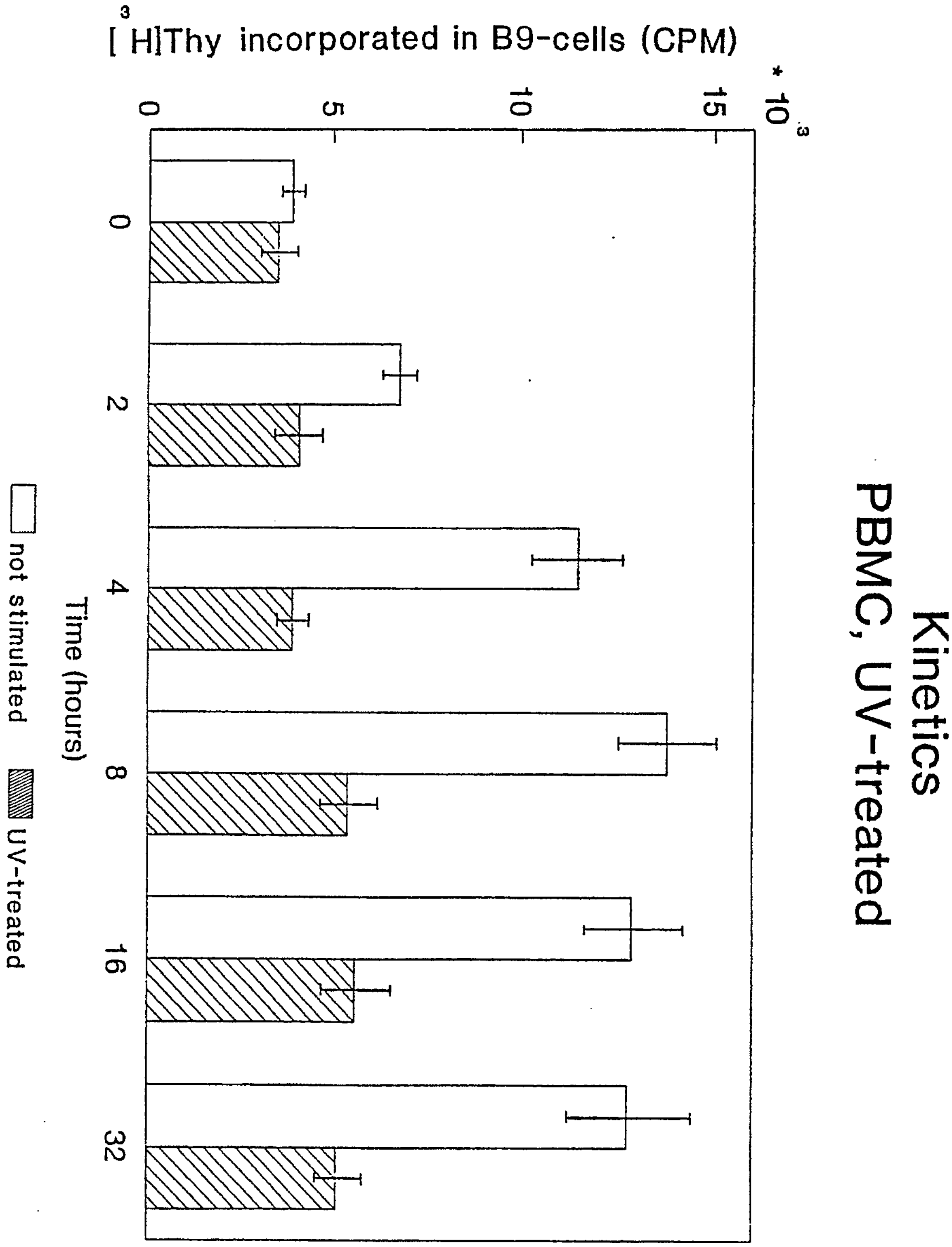
Kinetics
PBMC, UV-treated
[ 3H]Thy incorporated in B9-cells (CPM)
* 10 3
0
5
10
15
0
2
4
8
16
32
Time (hours)
not stimulated
UV-treated

Figure 4

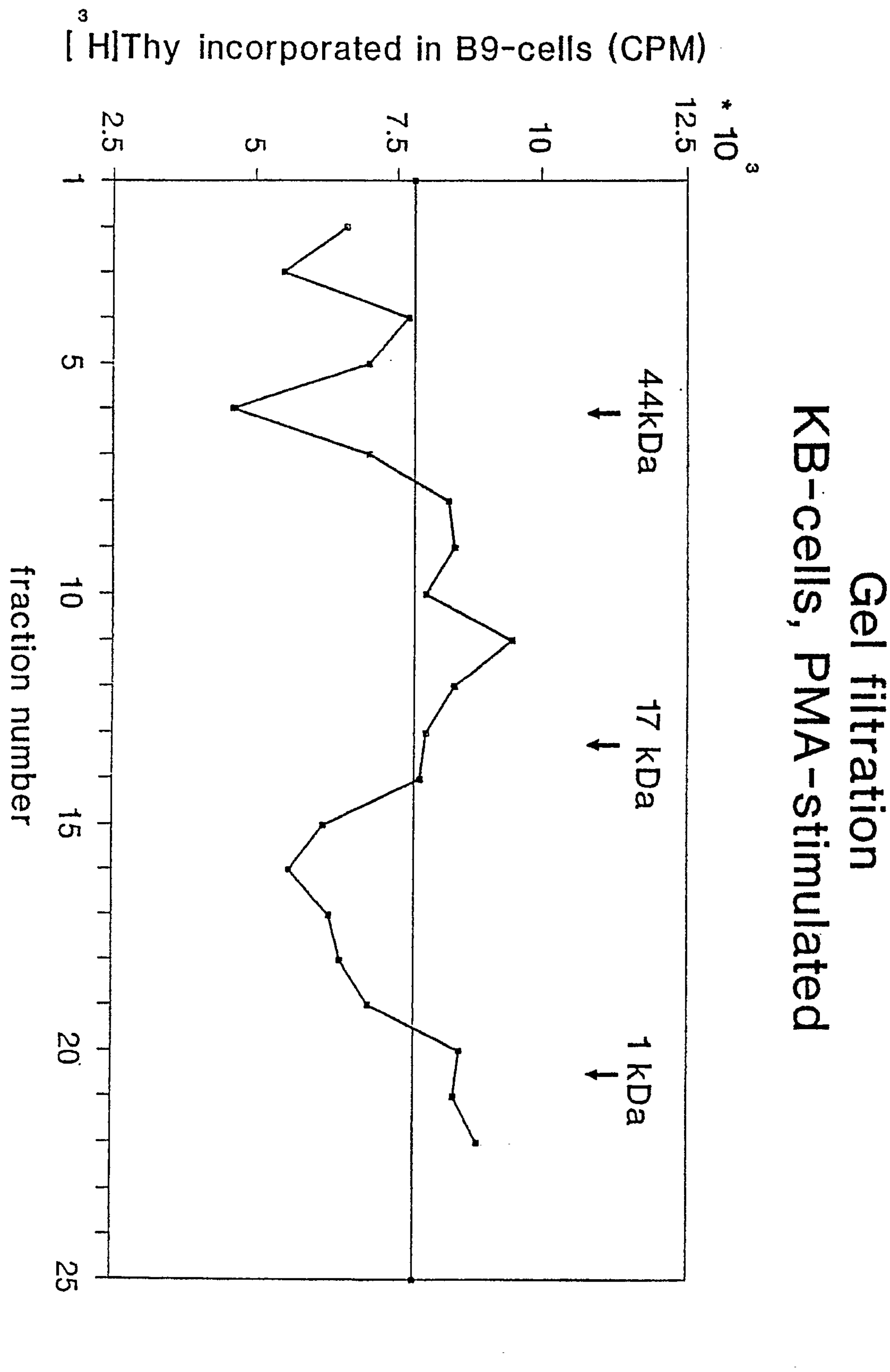
Gel filtration
KB-cells, PMA-stimulated
[$^3$H]Thy incorporated in B9-cells (CPM)
* 10$^3$
12.5
10
7.5
5
2.5
44kDa
17 kDa
1 kDa
1
5
10
15
20
25
fraction number

Figure 5

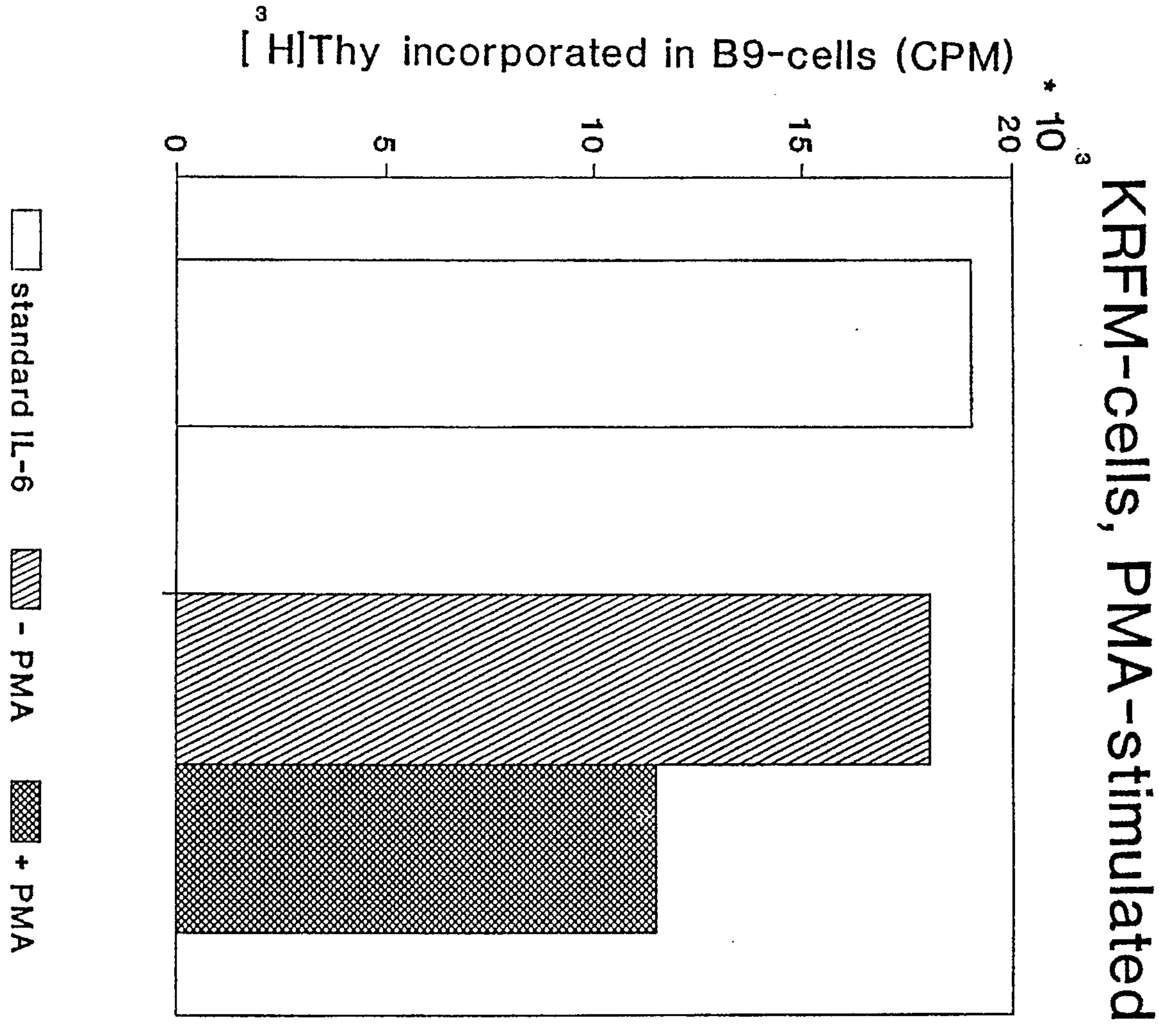
KRFM-cells, PMA-stimulated
[3H]Thy incorporated in B9-cells (CPM)
* 10 3
0
5
10
15
20
standard IL-6
- PMA
+ PMA

Figure 6

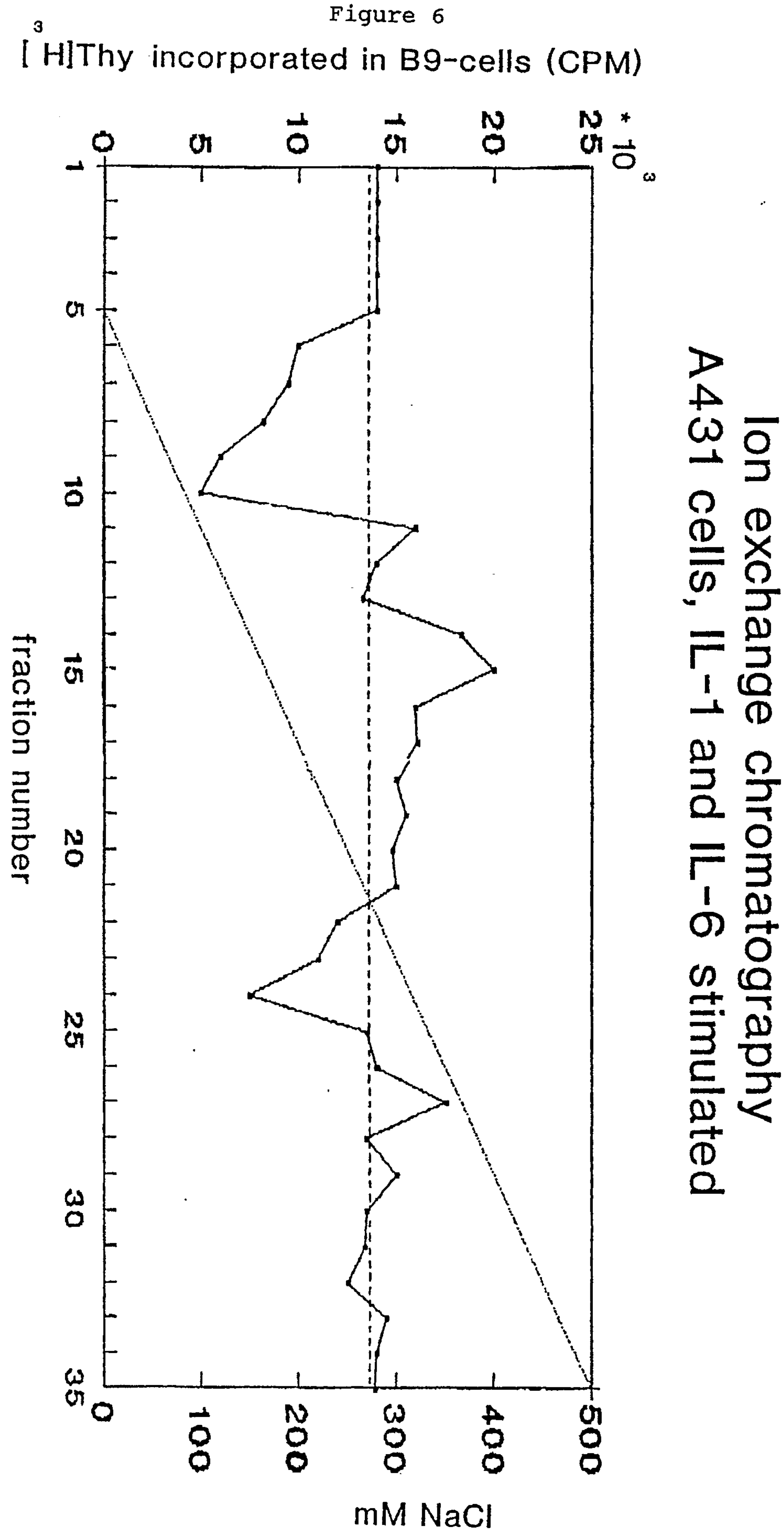
[3H]Thy incorporated in B9-cells (CPM)
* 10 3
Ion exchange chromatography
A431 cells, IL-1 and IL-6 stimulated
fraction number
mM NaCl

Figure 7

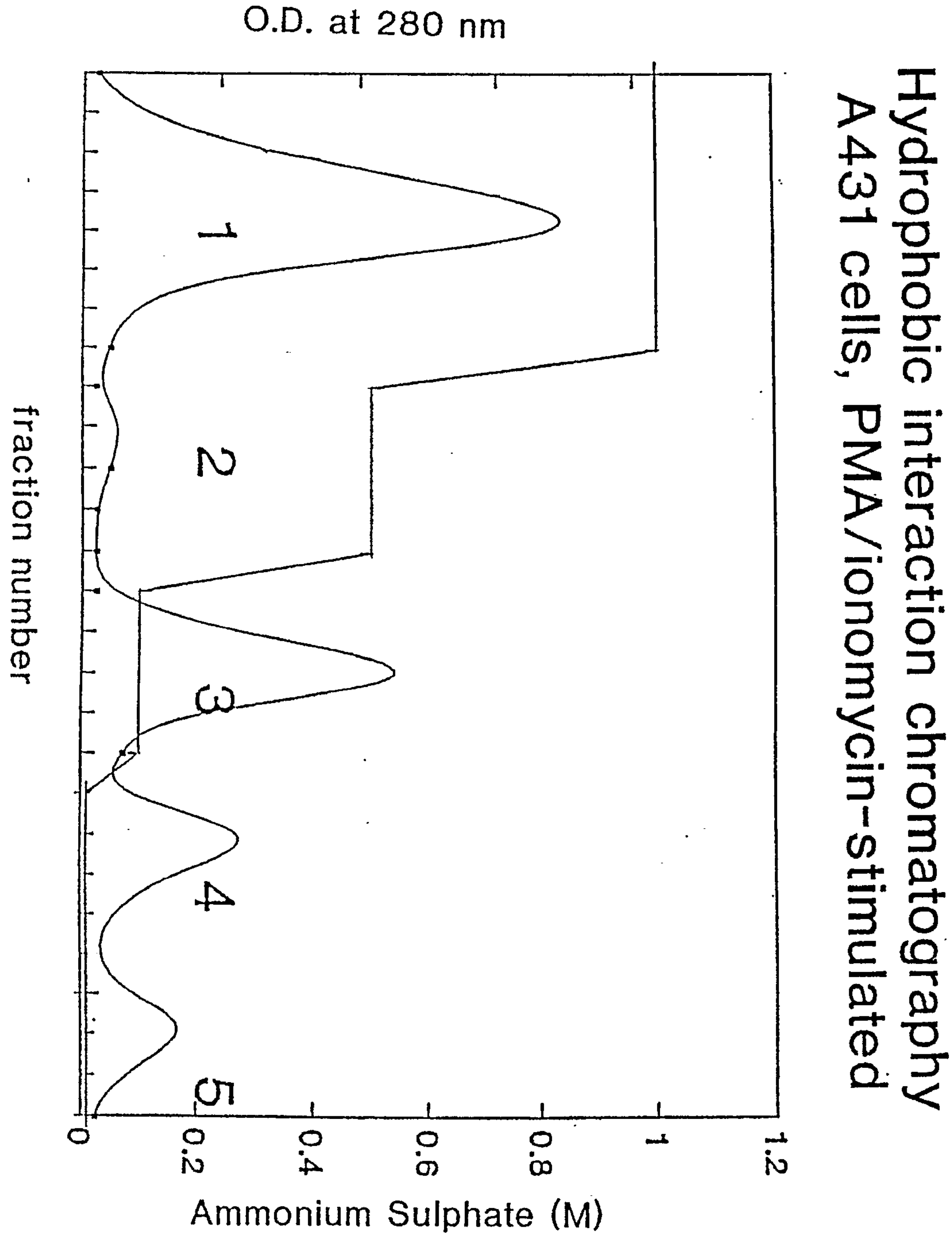
Hydrophobic interaction chromatography
A431 cells, PMA/ionomycin-stimulated
O.D. at 280 nm
fraction number
Ammonium Sulphate (M)
0
0.2
0.4
0.6
0.8
1
1.2
1
2
3
4
5

Figure 8

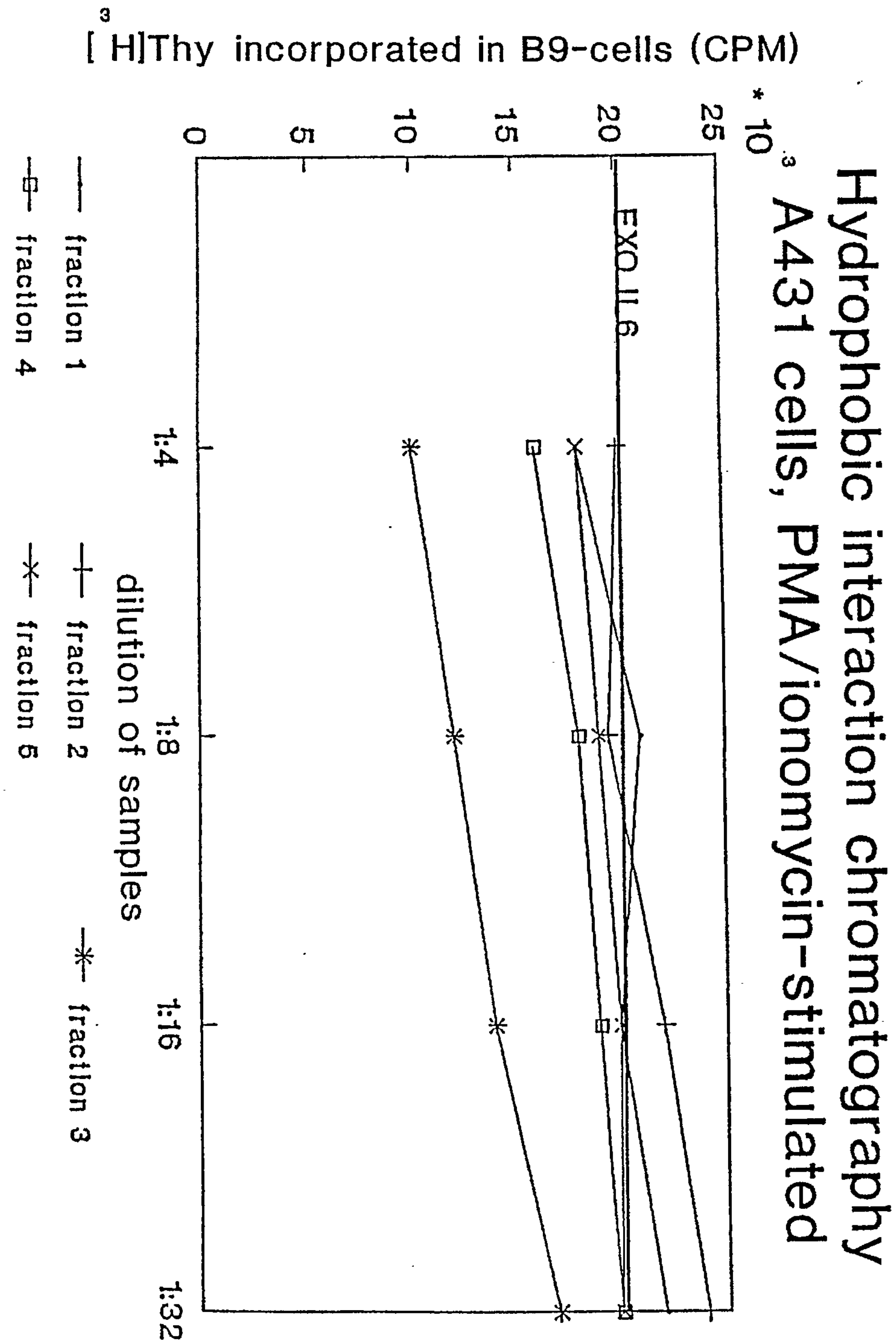
Hydrophobic interaction chromatography
A431 cells, PMA/ionomycin-stimulated
[3H]Thy incorporated in B9-cells (CPM)
* 10 3
0
5
10
15
20
25
EXO IL6
1:4
1:8
1:16
1:32
dilution of samples
fraction 1
fraction 2
fraction 3
fraction 4
fraction 5

Figure 9

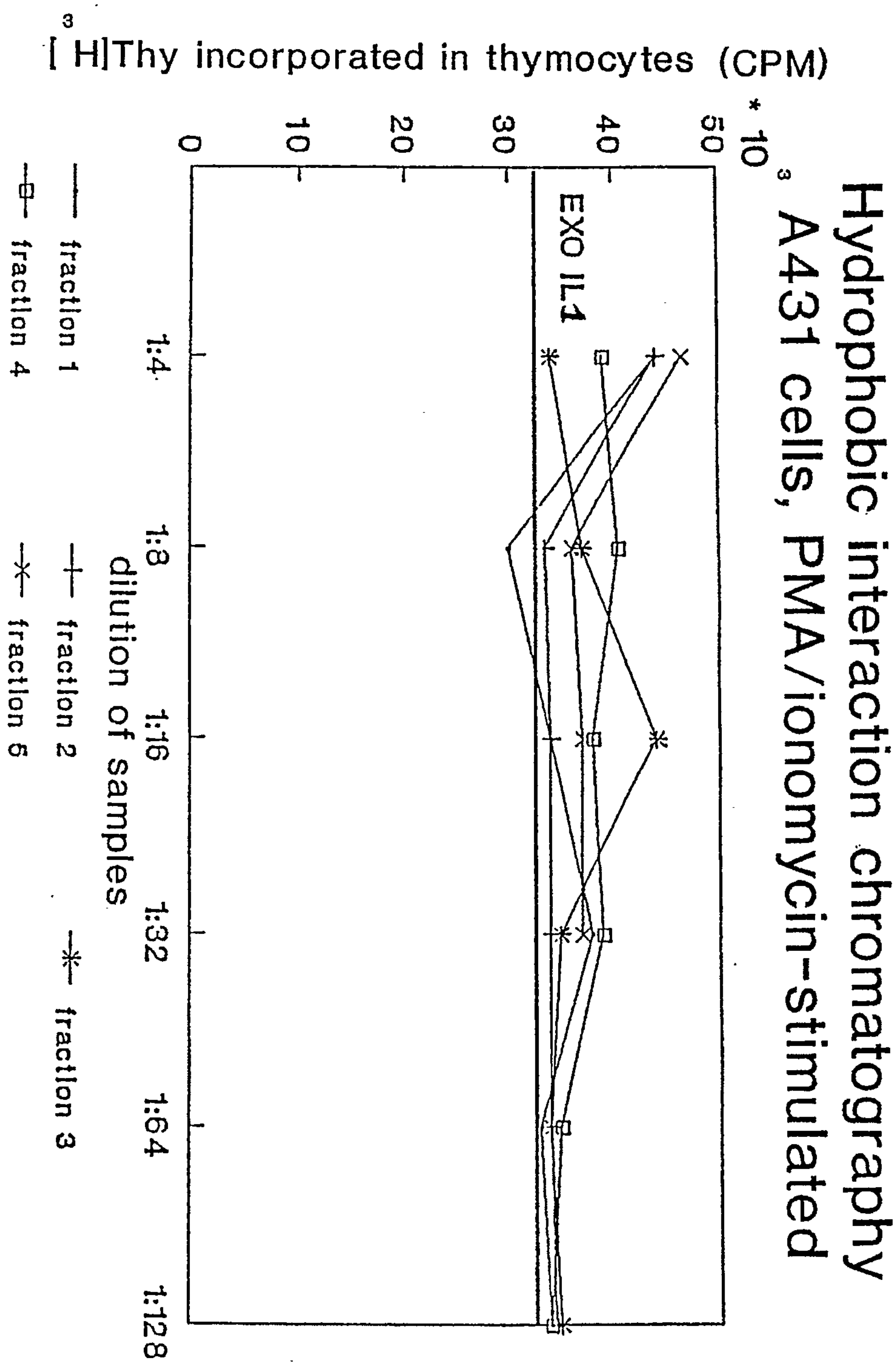
Hydrophobic interaction chromatography
A431 cells, PMA/ionomycin-stimulated
[3 H]Thy incorporated in thymocytes (CPM)
* 10 3
0
10
20
30
40
50
EXO IL1
1:4
1:8
1:16
1:32
1:64
1:128
dilution of samples
fraction 1
fraction 2
fraction 3
fraction 4
fraction 5

Figure 10

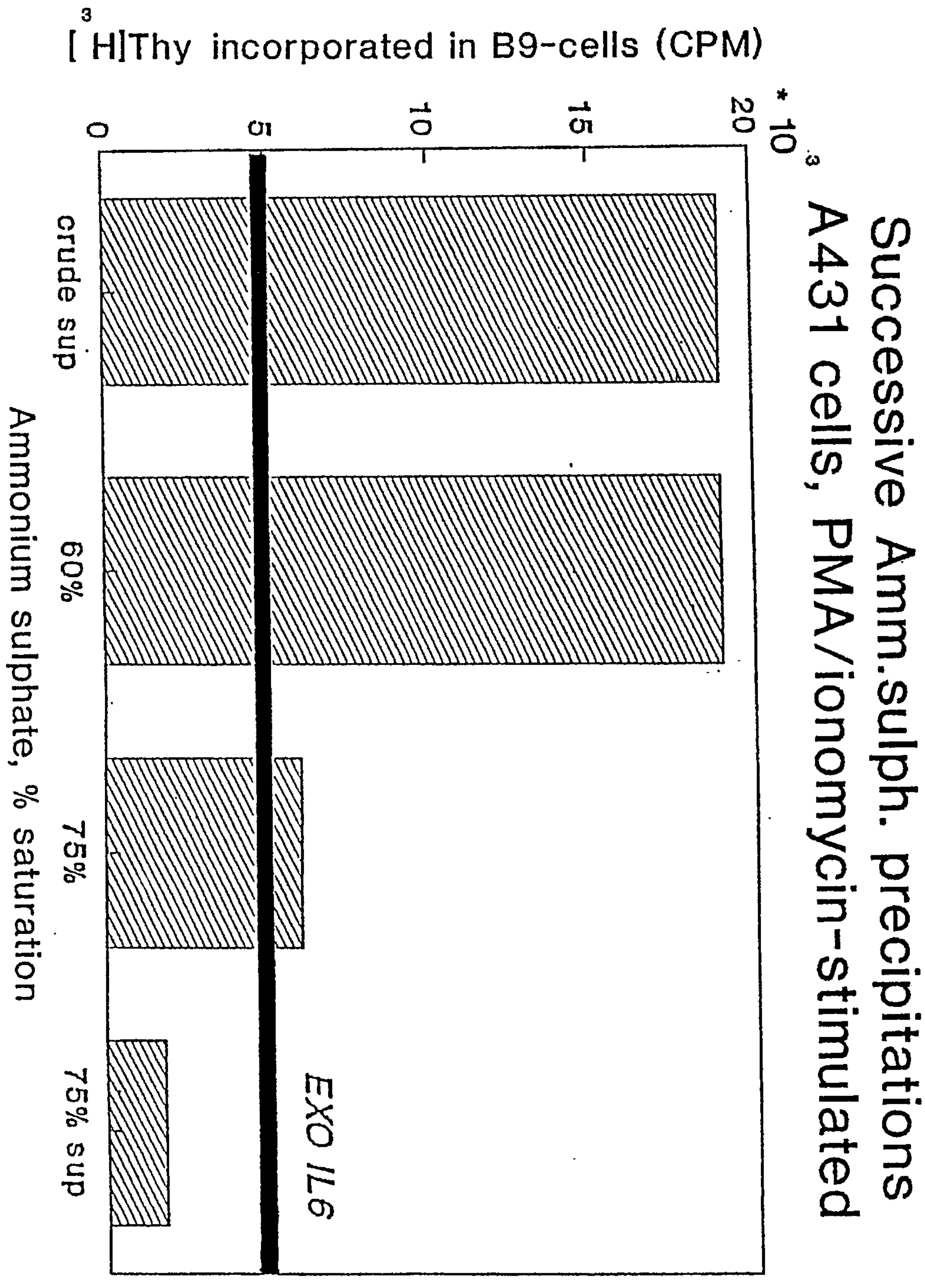
[3H]Thy incorporated in B9-cells (CPM)
* 10 3
0
5
10
15
20
Successive Amm.sulph. precipitations
A431 cells, PMA/ionomycin-stimulated
crude sup
60%
75%
75% sup
EXO IL6
Ammonium sulphate, % saturation

Figure 11

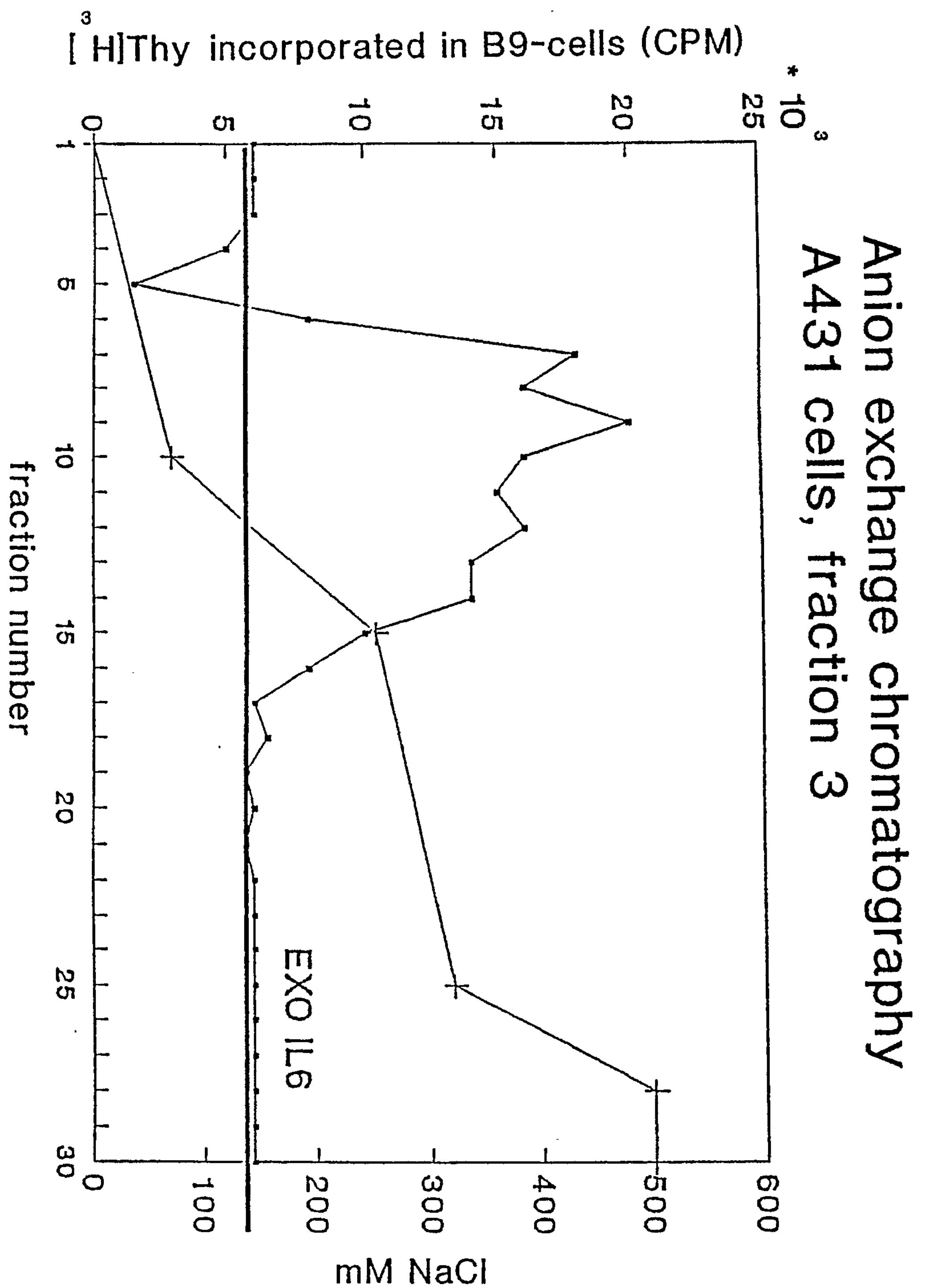
Anion exchange chromatography
A431 cells, fraction 3
[ $^3$H]Thy incorporated in B9-cells (CPM)
* 10 $^3$
0
5
10
15
20
25
fraction number
1
5
10
15
20
25
30
EXO IL6
mM NaCl
0
100
200
300
400
500
600

Figure 12

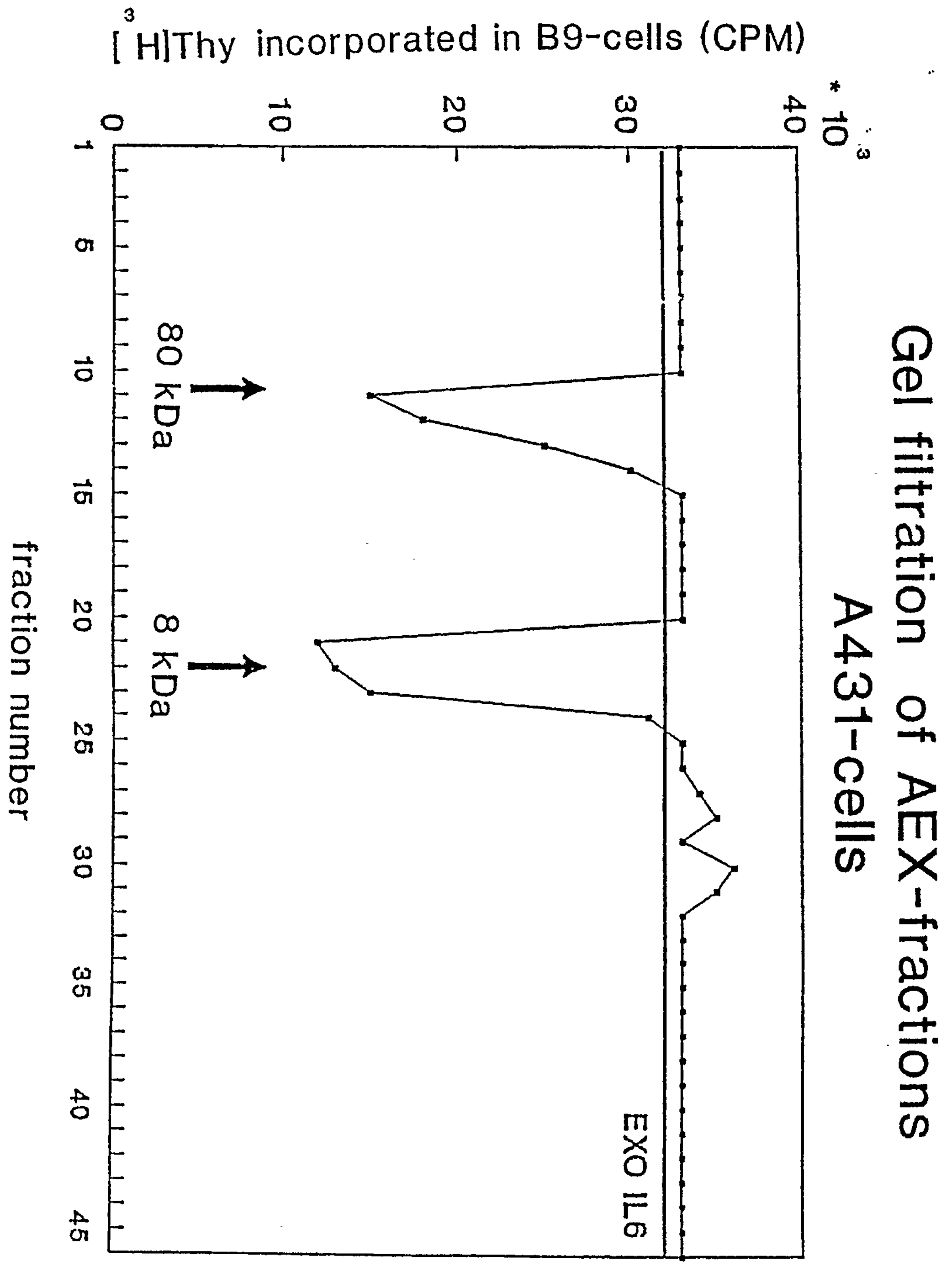
Gel filtration of AEX-fractions
A431-cells
[3H]Thy incorporated in B9-cells (CPM)
* 10 3
0
10
20
30
40
80 kDa
8 kDa
EXO IL6
1
5
10
15
20
25
30
35
40
45
fraction number

Figure 13

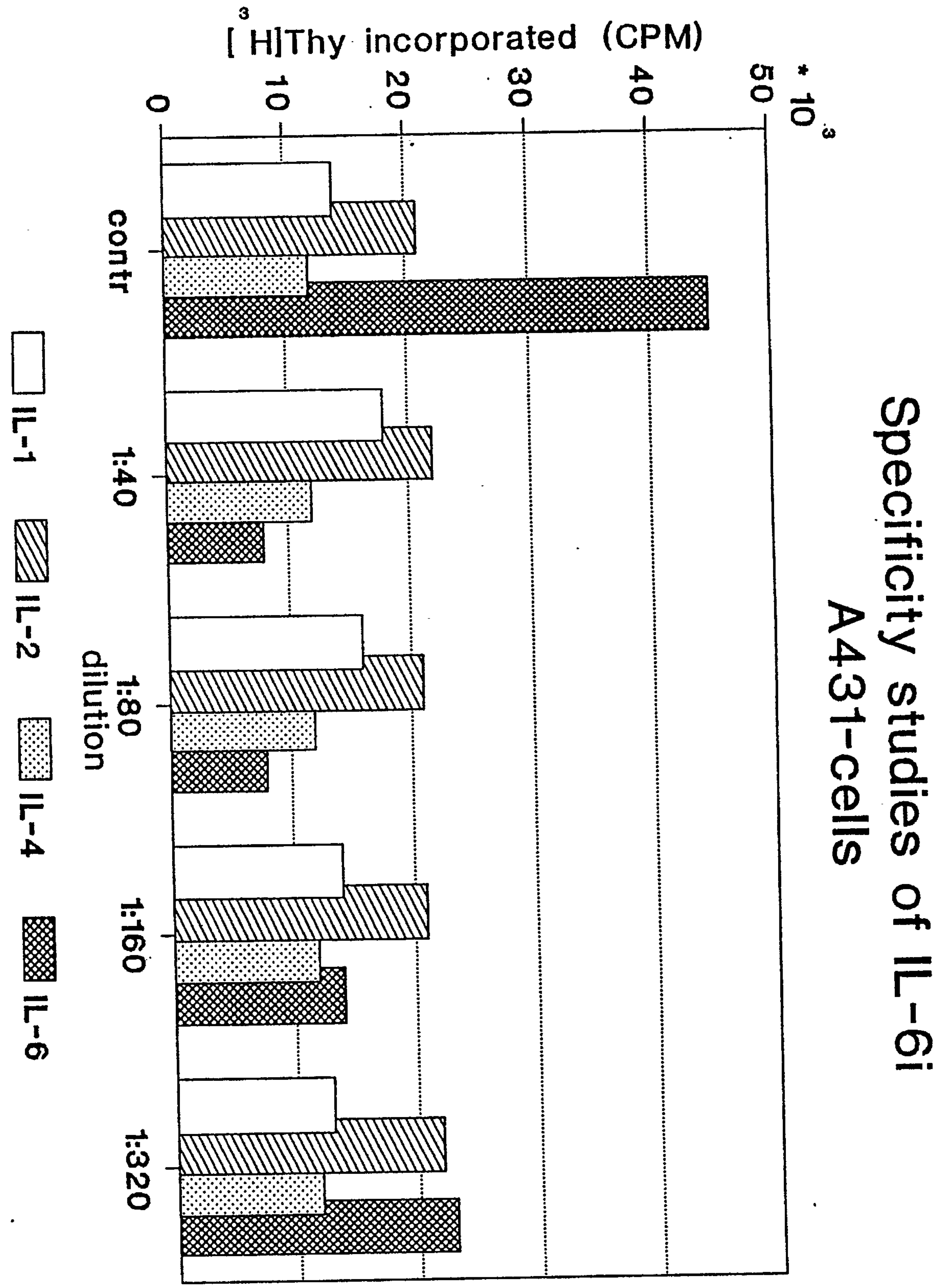
Specificity studies of IL-6i
A431-cells
[3H]Thy incorporated (CPM)
* 10 3
0
10
20
30
40
50
contr
1:40
1:80
dilution
1:160
1:320
IL-1
IL-2
IL-4
IL-6

Figure 14

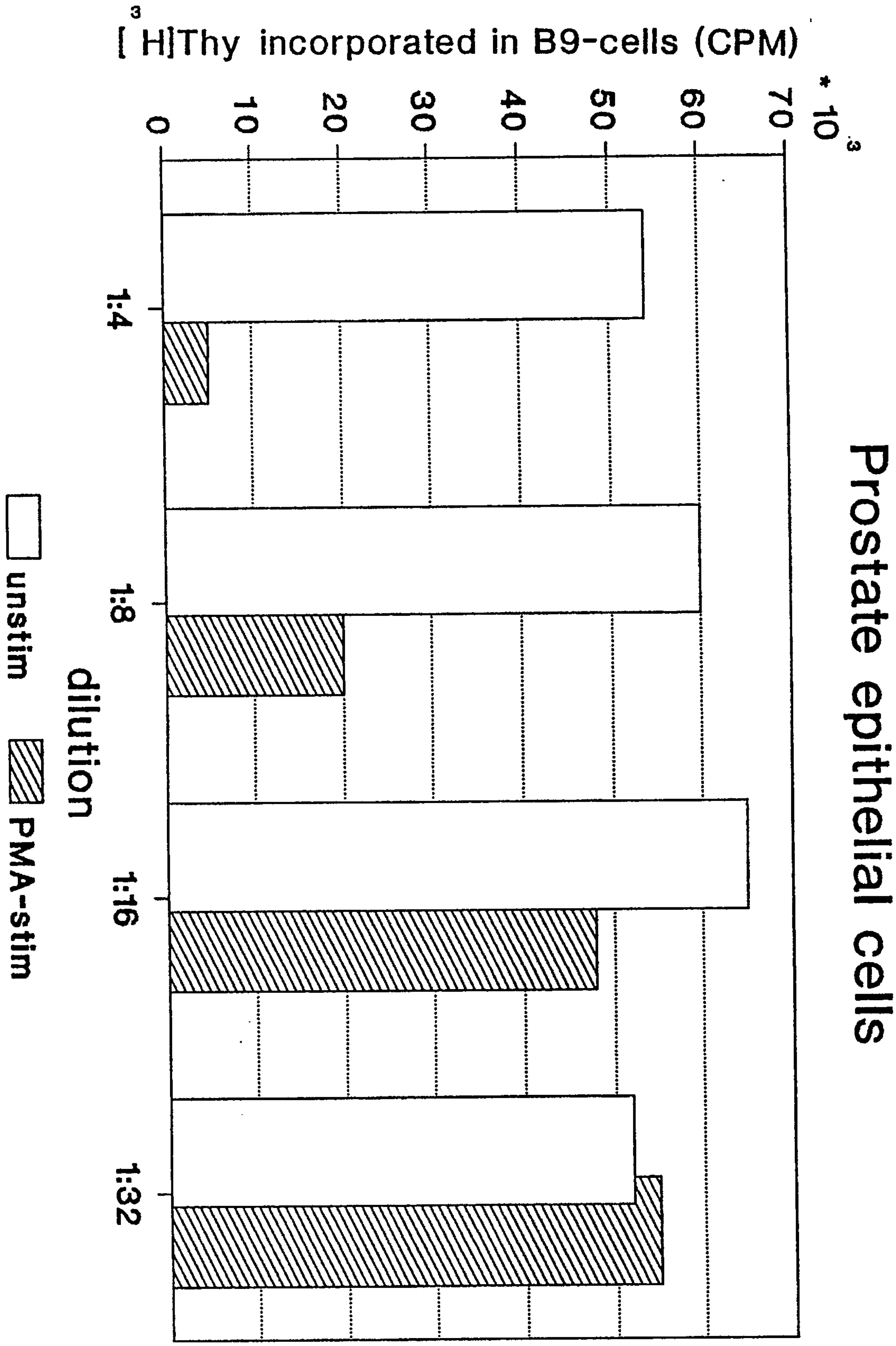
Prostate epithelial cells
[3H]Thy incorporated in B9-cells (CPM)
* 10 3
0
10
20
30
40
50
60
70
1:4
1:8
1:16
1:32
dilution
unstim
PMA-stim

Figure 15

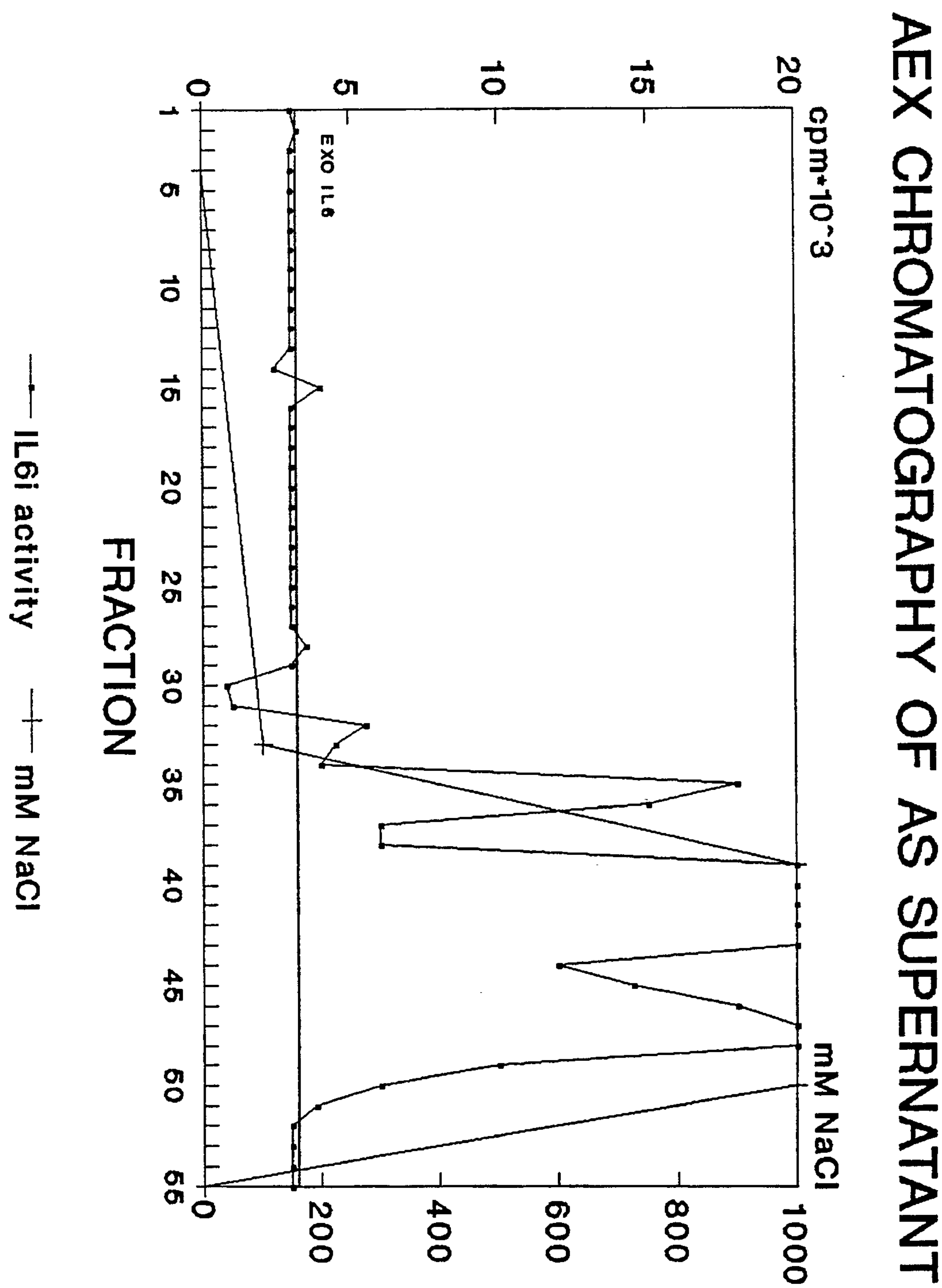
AEX CHROMATOGRAPHY OF AS SUPERNATANT
cpm*10^3
mM NaCl
EXO IL6
FRACTION
IL6i activity
mM NaCl
0
5
10
15
20
1
5
10
15
20
25
30
35
40
45
50
55
0
200
400
600
800
1000

Figure 16

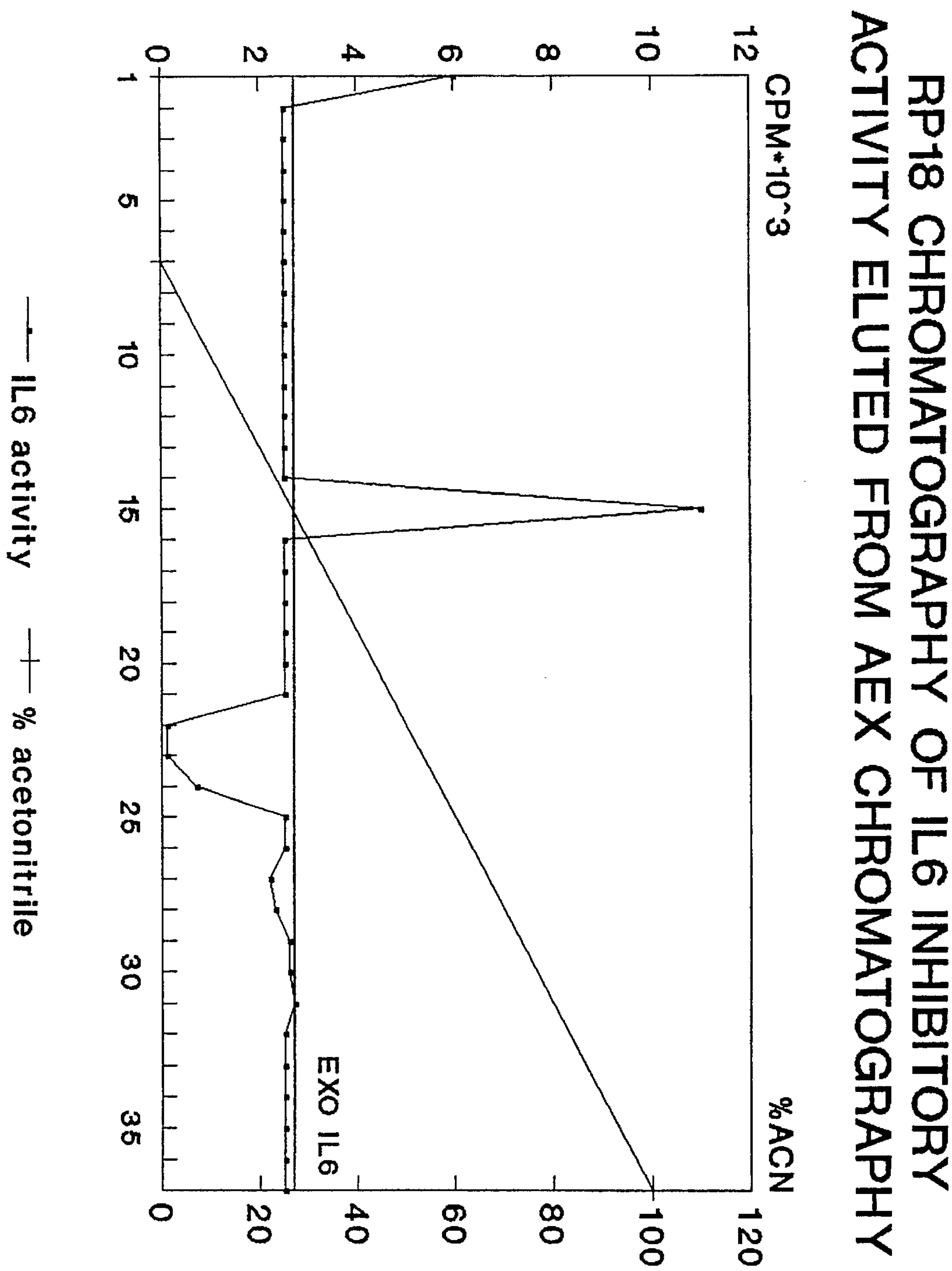
RP18 CHROMATOGRAPHY OF IL6 INHIBITORY
ACTIVITY ELUTED FROM AEX CHROMATOGRAPHY
CPM*10^3
%ACN
EXO IL6
0
2
4
6
8
10
12
1
5
10
15
20
25
30
35
0
20
40
60
80
100
120
IL6 activity
% acetonitrile

Figure 17

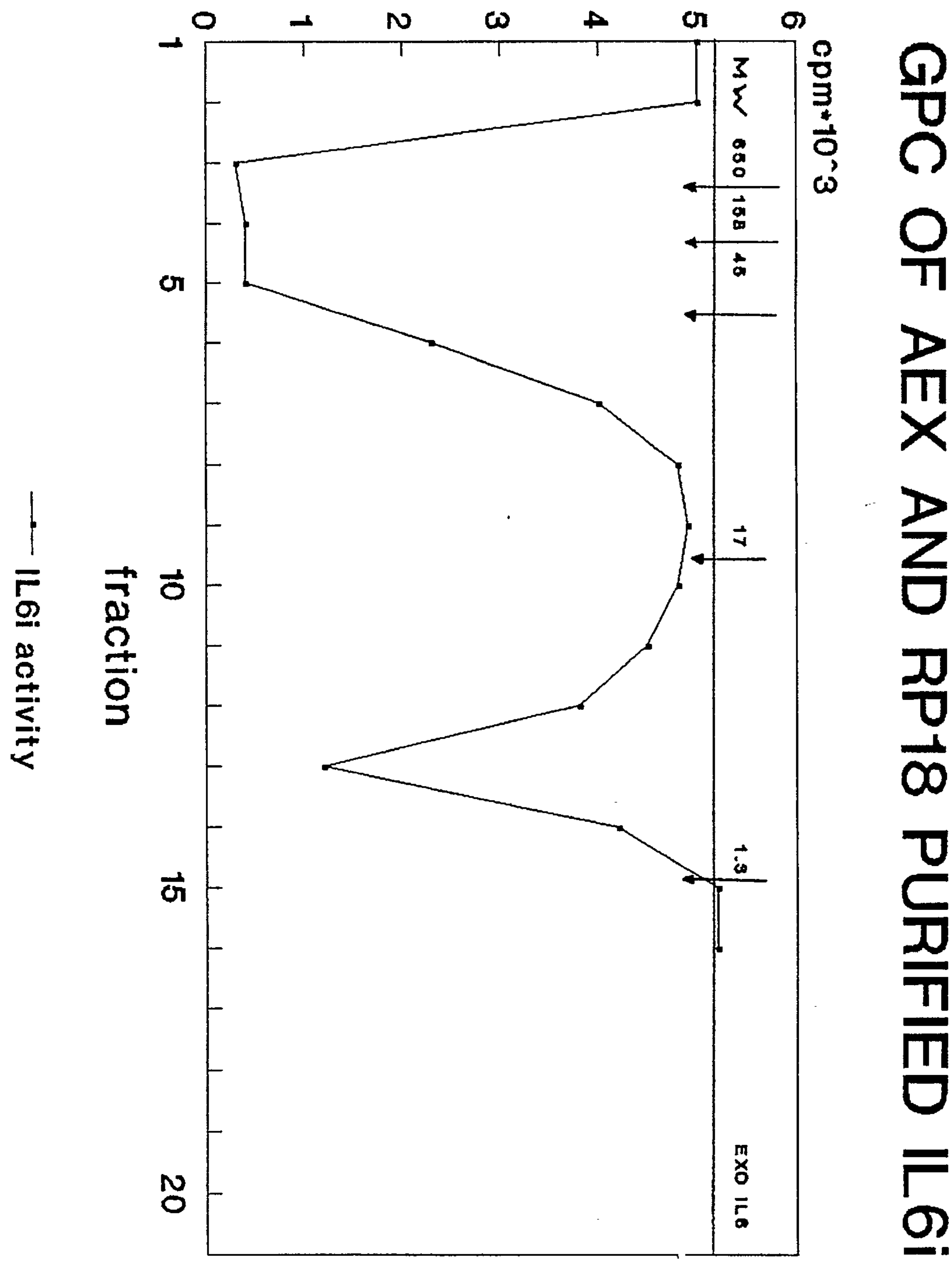
GPC OF AEX AND RP18 PURIFIED IL6i
cpm*10^3
MW 650 158 45
17
1.3
EXO IL6
0
1
2
3
4
5
6
1
5
10
15
20
fraction
IL6i activity